Europäisches Patentamt

European Patent Office

Office européen des brevets

(19)

(11) Numéro de publication: **0 085 629**
**B1**

(12) **FASCICULE DE BREVET EUROPEEN**

(45) Date de publication du fascicule du brevet: **06.04.88**

(51) Int. Cl.⁴: **B 01 D 21/00, C 02 F 1/52**

(21) Numéro de dépôt: **83400209.9**

(22) Date de dépôt: **31.01.83**

(54) **Procédé pour floculer des particules microscopiques en suspension dans un liquide et applications à la collecte des microalgues phytoplanctoniques et du zooplancton et à l'épuration d'eaux usées.**

(30) Priorité: **01.02.82 FR 8201589**

(43) Date de publication de la demande:
**10.08.83 Bulletin 83/32**

(45) Mention de la délivrance du brevet:
**06.04.88 Bulletin 88/14**

(84) Etats contractants désignés:
**AT BE CH DE FR GB IT LI LU NL SE**

(56) Documents cités:
**AT-B- 337 106**
**FR-A- 325 957**
**FR-A-1 501 912**
**GB-A-1 264 782**
**US-A-4 125 467**

(73) Titulaire: **Gozal, David**
**Centro Comercial Aplauso Mayor 44**
**Madrid 13 (ES)**

(72) Inventeur: **Elmaleh, Samuel**
**Les Olivettes 14 Clapiers**
**F-34170 Castelnau-le-Lez (FR)**
Inventeur: **Grasmick, Alain**
**Les Aires-7, rue des Aires**
**Montferrier/Lez (FR)**

(74) Mandataire: **Jolly, Jean-Pierre et al**
**Cabinet BROT et JOLLY 83, rue d'Amsterdam**
**F-75008 Paris (FR)**

Courier Press, Leamington Spa, England.

**0 085 629**

## Description

L'invention concerne un procédé pour floculer des particules microscopiques en suspension dans un liquide. L'invention concerne également l'application de ce procédé à la collecte des particules vivantes en suspension dans les eaux douces, saumâtres ou salées, notamment des microalgues constituant le phytoplancton et des animaux microscopiques constituant le zooplancton. L'invention concerne enfin l'application de ce procédé à l'épuration des eaux usées.

Les microalgues présentent un important intérêt économique, car leurs cellules contiennent des molécules de grande valeur, telles que des substances médicamenteuses, des hydrocarbures (glycérol, dans Dunaliella des marais salants), des protéines pouvant convenir à l'alimentation animale ou humaine; elles peuvent servir aussi de substrat pour une fermentation anaérobie produisant du méthane, gaz combustible.

C'est dans les bassins de lagunage destinés à l'épuration des eaux usées que l'on trouve les concentrations les plus élevées en microalgues susceptibles d'être rencontrées dans la nature (200 à 600 mg/l de produit sec avec $2.10^6$ à $4.10^6$ celleles algales par centimètre cube). Le processus d'épuration s'accompagne donc d'une importante production primaire de microalgues estimée entre 50 et 150 tonnes/ha/an. Ce phytoplancton peut être utilisé pour produire du zooplancton en aquaculture (essentiellement rotifères, copépodes et daphnies). Ces groupes d'animaux constituent une séquence alimentaire adéquate pour la nourriture des juvéniles de crustacés et de poissons, mais la production de cette séquence alimentaire, par les techniques actuelles, est coûteuse, sophistiquée et délicate à maîtriser.

En fait, le problème le plus délicat posé par ces techniques, qui constituent une forme d'exploitation de l'énergie solaire, est la collecte des microalgues.

On illustrera les difficultés d'ordre économique que soulève cette collecte, en se référant à la technique de méthanisation des microphytes, qui, dans le domaine de la production d'énergie renouvelable, soulève un vif intérêt depuis quelques années.

Dans le cas le plus favorable -contrôle des espèces collectées, récolte par des techniques nécessitant les plus faibles puissances, absence de séchage-, le contenu énergétique brut de la production algale (quantité totale d'enthalpie utilisée pour maintenir le système de production en fonctionnement à partir des matières premières naturelles) est de l'ordre de 18 MJ/kg. Par fermentation méthanogène, on peut récupérer 60% du pouvoir calorifique inférieur -chaleur recueillie par combustion, l'eau formée étant à l'état vapeur- ce pouvoir calorifique s'élevant à 23,2 MJ/kg, soit 13,9 MJ/kg. Ceci signifie, par exemple, qu'une masse de 80 kg de microphytes permet de produire 1 GJ par méthanisation, mais que sa production ayant nécessité 1,44 GJ, le bilan énergétique global est négatif, du fait, essentiellement, de la complexité de la séparation des microphytes du milieu aqueux.

En raison de leurs dimensions (10—30 µm) et de leurs caractéristiques propres (organismes vivants), les algues microscopiques posent, en effet, de difficiles problèmes de séparation. Certains procédés déjà testés se sont révélés ou peu performants ou gros consommateurs d'énergie (c'est le cas de la décantation centrifuge ou de la flottation, testées à grande échelle pour la séparation du plancton).

Dans le traitement de potabilisation des eaux, le microtamisage est utilisé avec succès depuis de nombreuses années, avec pour objectif prioritaire l'élimination des algues. Les matières en suspension éliminées sont récupérées dans un état plus concentré, mais sous forme d'un liquide encore relativement peu chargé (taux de concentration: 20 à 30).

Avec la filtration sur matériau granulaire, on obtient de meilleurs résultats, mais au prix d'une plus grande consommation d'énergie. Là encore, une récupération des matières en suspension retenues est envisageable avec un taux de concentration de 20 à 30.

Les performances de certains des procédés cités peuvent être améliorées par adjonction de réactifs chimiques permettant de floculer les algues. Mais cette opération augmente considérablement les coûts de production et peut compromettre l'utilisation du produit dans une séquence alimentaire.

En conséquence, si une suspension concentrée de quelques grammes à quelques dizaines de grammes par litre présente un intérêt, des procédés tels que le microtamisage ou la filtration peuvent s'appliquer. Si une concentration plus importante doit être atteinte, il n'existe pas jusqu'à ce jour de procédé répondant à la fois aux exigences techniques et économiques.

FR—A—1 501 912 décrit, par exemple, un procédé pour clarifier de l'eau chargée d'impuretés en suspension, selon lequel on ajoute à l'eau un additif choisi dans le groupe des floculants ou des coagulants, on leu fait ensuite traverser, de bas en haut, dans un réservoir, un lit fluidisé de matière granuleuse, à une vitesse an moins égale à celle nécessaire au maintien du lit en état de fluidisation, on ralentit la vitesse de l'eau au sommet du réservoir et l'on recueille le liquide traité. Ce procédé nécessite donc l'emploi de réactifs. En outre, il est indiqué dans ce brevet qu'une partie des grains du lit fluidisé est entraînée par l'eau traitée et l'on doit ensuite les séparer.

La présente invention vise à remédier à ces inconvénients en proposant un procédé pour floculer les microalgues ou autres particules organiques ayant des propriétés voisines sand adjonction de réactifs, moyennant une faible consommation d'énergie, et avec une modification des propriétés physiques de ces microalgues telle qu'une simple décantation permette de dépasser des taux de concentration de l'ordre de 100 et ce, sans altérer leurs qualités nutritionnelles.

On rappelle que la floculation est un phénomène complexe, qui aboutit à la formation de flocons, c'est-

2

à-dire d'une structure réticulée et poreuse. On confond souvent sous cette dénomination les phénomènes de coagulation et de floculation vraie, qui ont pour effet de créer des agrégats facilement séparables. A titre d'exemple, le traitement physico-chimique des eaux usées de toute origine comporte une phase de floculation, qui est obtenu par addition à ces eaux d'un réactif approprié (chlorure ferrique, sulfate d'aluminium, polyélectrolytes ou autres) et qui est suivie d'une phase de séparation liquide-solide par décantation.

Cette clarification par floculation est particulièrement utile dans le traitement des eaux usées à débit très variable, excluant l'emploi des procédés biologiques traditionnels. En revanche, ce type de traitement présente le grave inconvénient d'être d'un coût élevé, du fait de la consommation des agents de floculation.

L'inventeur a conçu un procédé pour provoquer la floculation de particules microscopiques en suspension dans un liquide, qui peut être appliqué avantageusement à la collecte de microorganismes vivants et qui ne requiert pas l'addition de réactifs de floculation.

Ce procédé est basé sur le fait que des particules microscopiques peuvent s'agglomérer et floculer spontanément dans certaines conditions (voir, par exemple, "Biotechnology and Exploitation of Algae. The Indian Approach (1973—1981)", pour la floculation des microalgues; voir également "Wastewater Treatment" de Donald W. SUNDSTROM et Herbert E. KLEI, Prentice-Hall, Inc. 1979, pages 196—199, pour la floculation des eaux usées).

L'invention a par conséquent pour objet un procédé pour floculer en continu, sans adjonction de réactifs, des particules microscopiques telles que des microalgues (c'est-à-dire des algues de dimensions entre 10 et 30 μm) en suspension dans un liquide, caractérisé en ce que l'on fait passer un premier courant du liquide comprenant les particules microscopiques en suspension à travers un milieu granulaire jusqu'à colmatage partiel ou complet dudit milieu granulaire par lesdites particules microscopiques et en ce que l'on décolmate au moins partiellement ledit milieu granulaire en le faisant traverser par un second courant du liquide à traiter, circulant à une vitesse par unité d'aire de la section transversale supérieure à celle du premier courant et comprise entre 0,25 et 1 m/h, en récupérant en aval du milieu granulaire, dans le sens de passage desdits courants de liquides, des agrégats floculés desdites particules microscopiques et un effluent liquide, partiellement ou totalement épuré.

Le milieu granulaire pourra demeurer en lit fixe ou subir une expansion d'au plus environ 30% en volume, le milieu granulaire se trouvant alors à l'état fluidisé.

Le milieu granulaire pourra avantageusement être constitué de particules de granulométrie comprise entre environ 0,005 et 3 cm et de masse volumique d'environ 1,1 g/cm⁶ ou davantage. Les dimensions exactes et la nature des particules seront choisies en fonction de la suspension traitée et du résultat désiré. Comme milieu granulaire, on pourra utiliser simplement du sable naturel. On peut aussi employer d'autres particules, à angles vifs ou arrondis, par exemple des céramiques ou des particules d'argile calcinée et broyée.

Avantageusement, ce procédé pourra être mis en oeuvre dans une colonne verticale ou inclinée, cylindrique ou hélicoïdale, contenant le milieu granulaire. Cette colonne sera alimentée à sa base par le liquide à traiter, tandis que les agrégats de particules floculées seront récupérés à un niveau supérieur à celui du milieu granulaire et que l'effluent de liquide épuré sera évacué à un niveau encore supérieur. Bien que cette forme de mise en oeuvre du procédé conforme à l'invention soit préférée, on peut cependant appliquer ce procédé en flux descendant.

De préférence, pour favoriser la concentration de floculat, la partie de la colonne où celui-ci sera récupéré, en flux ascendant, aura une section de surface supérieure à celle de la partie contenant le milieu granulaire.

Dans son application à la collecte de microalgue ou de zooplancton, le procédé conforme à l'invention comprendra au moins une phase additionnelle de concentration des floculats séparés du milieu granulaire, par exemple par décantation, éventuellement, comme il vient d'être indiqué, à l'intérieur même de la colonne dans laquelle le procédé est mis en oeuvre. On pourra aussi utiliser d'autres moyens de concentration connus dans la technique, que l'on ne peut utiliser habituellement pour collecter des microalgues ou du zooplancton en suspension, du fait de l'infime concentration de ces suspensions.

Dans son application à la purification et à la clarification des eaux usées, le procédé conforme à l'invention ne nécessitera pas de réactifs de floculation et sera donc beaucoup moins coûteux que les procédés usuels comportant une phase de floculation.

Les figures 1 et 2 des dessins annexés sont des schémas illustrant deux formes de mise en oeuvre préférées du procédé conforme à l'invention.

La figure 3 illustre une autre forme de mise en oeuvre de ce procédé.

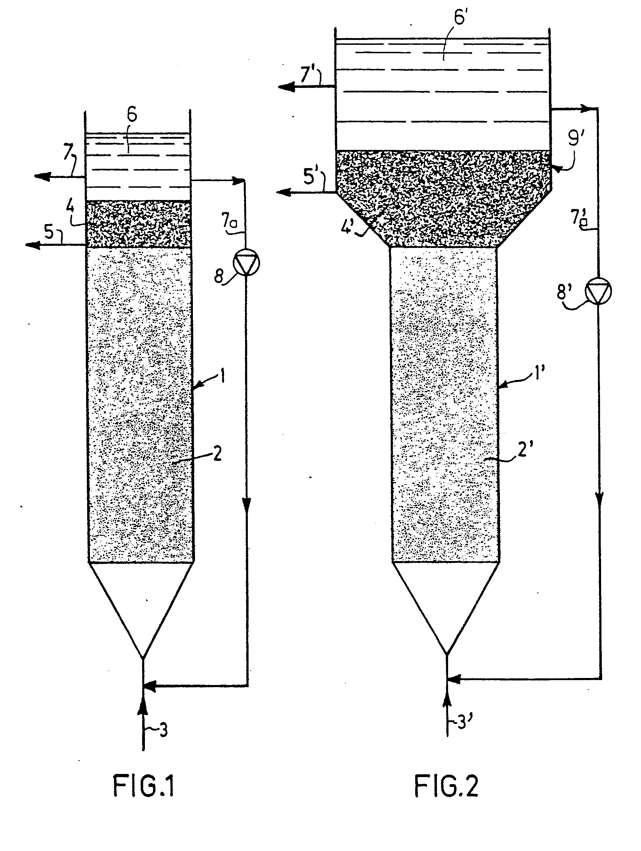

On se référera d'abord à la figure 1, sur laquelle on voit une colonne 1, dans laquelle est logé un lit granulaire 2 d'un matériau dont la nature, la masse volumique et la granulométrie sont choisies en fonction du résultat désiré et de la suspension à traiter. Il pourra être constitué, par exemple, de sable naturel (quartz et quartzites) de granulométrie comprise entre 0,2 et 4 mm.

Le lit granulaire 2 est alimenté en liquide par une ligne 3 connectée à la base de la colonne.

Dans un premier temps, on alimente le lit granulaire 2 par la ligne 3 avec un premier courant de liquide contenant des particules microscopiques en suspension, et l'on procède ainsi à une filtration de ce liquide à travers un lit fixe de particules solides. On provoque par conséquent un colmatage progressif du lit 2 et l'on

**0 085 629**

constate une augmentation de la perte de charge à travers ce lit. On arrête l'alimentation lorsque cette perte de charge atteint une valeur prédéterminée, correspondant à un colmatage partiel du lit 2, ou éventuellement après colmatage complet de ce lit. Dans des essais effectués par l'inventeur, la perte de charge correspondant à un colmatage satisfaisant du lit 2 a été de l'ordre de 50 cm de hauteur d'eau par mètre de hauteur du lit granulaire.

Le lit granulaire 2 ayant ainsi été traité, on peut l'utiliser pour provoquer la floculation en continu la suspension de particules microscopiques telles que des microalgues.

Un deuxième courant de la suspension à traiter est injecté par la ligne 3 à la base de la colonne 1 à une vitesse supérieure à celle de la suspension précédemment utilisée pour colmater le lit granulaire, et de l'ordre, généralement, de 0,1 à 5 m/h.

De façon surprenante, on observe, a la sortie du lit granulaire 2, la formation d'un floculat des particules microscopiques de la suspension. Les flocons peuvent avoir des dimensions comprises entre 1 et 5 mm et, par décantation, ils se séparent au sommet de la colonne 1 en formant une couche 4, qui peut être évacuée, par intermittence ou en continu, par une ligne 5. Cette couche 4 de floculat est surmontée d'une couche 6 de liquide au moins partiellement épuré, qui est évacué par la ligne 7. Une partie au moins de cet effluent liquide peut d'ailleurs être recyclée par une pompe 8 et par la ligne 7a à la ligne d'alimentation 3, afin de récupérer le floculat qui peut y être présent.

Le lit granulaire 2 ayant subi le prétraitement de colmatage partiel ou total peut ensuite être utilisé en continu pour floculer les particules microscopiques présentes dans la suspension d'alimentation. Le taux de concentration entre la suspension d'origine et le floculat atteint, par exemple, quelques dizaines dans le cas de microalgues.

La raison de ce phénomène de floculation n'est pas totalement élucidée, mais on suppose que le colmatage du lit granulaire et le décolmatage partiel, qui le suit ont pour effet de créer à l'intérieur de ce lit des cheminées qui constituent autant de trajets préférentiels qu'emprunte la suspension à traiter. La seule énergie nécessaire à la mise en oeuvre du procédé conforme à l'invention est celle nécessaire pour assurer la circulation du liquide à travers le lit et elle est donc très faible.

Suivant les conditions de mise en oeuvre du procédé, le lit 2 peut rester fixe ou subir une légère expansion, de l'ordre de 30%; la couche peut donc être fixe, préfluidisée ou fluidisée.

Le floculat évacué par la ligne 5 peut être très facilement concentré par les méthodes connues de séparation liquide-solide. Une simple décantation permet d'atteindre un taux de concentration de plusieurs centaines pour les microalgues. Le volume occupé par un gramme de matière sèche, après 10 mn de décantation en éprouvette, est de l'ordre de 70 cm$^3$, ce qui est un résultat particulièrement intéressant.

La figure 2 illustre une variante préférée de mise en oeuvre du procédé conforme à l'invention. Sur cette figure, les organes déjà décrits à propos de la figure 1 sont désignés par les mêmes chiffres de référence affectés de l'indice '.

La seule différence notable avec la figure 1 est que la colonne 1' contenant le lit granulaire 2' s'évase au-dessus de ce lit et est surmontée d'une colonne 9', dont la section transversale est supérieure à celle de la colonne 1'. Dans ces conditions, la vitesse ascensionnelle du liquide dans la colonne 9' est plus faible que dans la colonne 1', ce qui a pour effet de favoriser la concentration du floculat.

Les figures 1 et 2 illustrent une forme de mise en oeuvre préférée de l'invention, dans laquelle le lit granulaire 2 ou 2' est traversé par un flux ascendant de liquide à traiter.

L'invention peut tout fois être mise en oeuvre, également, avec un lit granulaire traversé de haut en bas par la suspension de particules microscopiques à floculer.

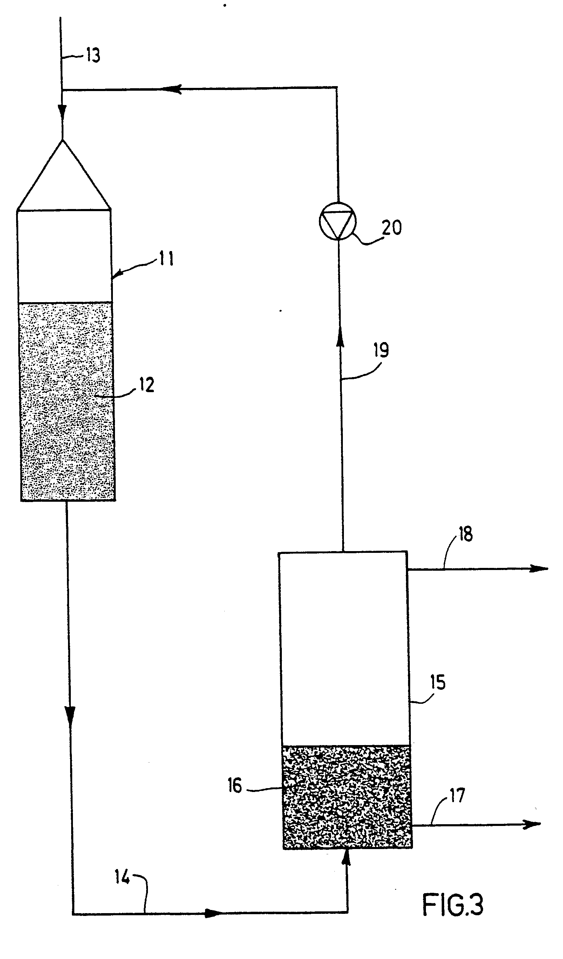

La figure 3 illustre cette forme de mise en oeuvre.

Dans cette variante, la colonne 11, contenant un lit granulaire 12, est alimentée par une ligne 13, à sa partie supérieure, en liquide contenant des particules microscopiques en suspension. L'effluent contenant les flocons de particules floculées est évacué à la base de la colonne 11, par une ligne 14, en direction de la partie inférieure d'un décanteur vertical 15. Dans ce décanteur 15, l'effluent de la colonne 11 s'élève à contre-courant du floculat 16 de particules microscopiques, qui se concentre à la partie inférieure du décanteur 15 et est évacué par une ligne 17. Le liquide partiellement épuré est évacué à la partie supérieure du décanteur 15 par une ligne 18 et une partie de ce liquide peut être éventuellement recyclée, comme précédemment, par une ligne 19 et une pompe 20 vers la ligne 13 d'alimentation de la colonne 11.

Les exemples suivants illustrent diverses applications du procédé conforme à l'invention.

## Exemple I

Cet exemple concerne l'application du procédé ci-dessus à la collecte de microalgues phytoplanctoniques en suspension dans l'eau.

Divers essais ont été effectués, avec différents types de lit granulaire, dans une colonne verticale à flux ascendant, avec des microalgues en suspension dans de l'eau.

Le Tableau suivant rassemble les résultats de ces essais et illustre l'efficacité du procédé conforme à l'invention.

4

0 085 629

TABLEAU

| Nature du granulaire | Granulométrie (mm) | Vitesse du flux en fût vide (m/h) | Expansion (%) | Concentration à l'entrée de la colonne (mg/l) | Concentration après floculation et décantation en éprouvette (10 mm) (mg/l) | Taux de concentration |
|---|---|---|---|---|---|---|
| Argile calcinée broyée | 3—5 | 1 | 0 | 200 | 50 000 | 250 |
| Céramique | 1—2 | 1 | 0 | 100 | 20 000 | 200 |
| Sable | 0,2—0,25 | 0,25 | 10 | 60 | 8 400 | 140 |
| | | 0,50 | 15 | 100 | 1 400 | 140 |
| | | 1 | 20 | 100 | 1 400 | 140 |

0 085 629

Ce tableau illustre l'efficacité du procédé conforme à l'invention et montre, en particulier, qu'un lit granulaire constitué d'argile calcinée broyée à granulométrie importante (3 à 5 mm) est, de façon surprenante, particulièrement avantageux, pour mettre en oeuvre ce procédé.

L'invention apporte donc un moyens simple, facile à mettre en oeuvre et peu coûteux, pour concentrer des suspensions à concentration très faible de particules microscopiques. Il est clair que, dans son application à la collecte des microalgues ou du zooplancton, ce procédé est particulièrement intéressant, car il n'altère en rien les propriétés physico-chimiques des microorganismes récupérés, puisque ceux-ci restent à l'état vivant dans leur milieu naturel, pendant toute la durée de la concentration et de la collecte.

Exemple II

Cet exemple concerne l'application du procédé conforme à l'invention à l'épuration d'eaux usées d'origine urbaine ayant subi une clarification préliminaire par simple décantation.

Ces eaux, présentant une concentration de matières en suspension variant entre 150 et 200 mg/l, traversent un lit fixe de sable d'une granulométrie de 300 μm, sans addition d'aucun réactif de floculation.

On note un taux d'expansion du lit de sable de 10%.

On constate que 50% des matières en suspension sont retenues pour un temps de passage d'une heure, tandis que 70% de ces matières sont retenues avec un temps de passage de trois heures.

Les matières en suspension retenues constituent un floculat qui se dépose à la surface du lit granulaire. Ce floculat peut être éliminé périodiquement ou en continu.

## Revendications

1. Procédé pour floculer en continu, sans adjonction de réactifs, des particules microscopiques telles que des microalgues (c'est-à-dire des algues de dimensions entre 10 et 30 μm) en suspension dans un liquide, caractérisé en ce que l'on fait passer un premier courant du liquide comprenant les particules microscopiques en suspension à travers un milieu granulaire (2) jusqu'à colmatage partiel ou complet dudit milieu granulaire par lesdites particles microscopiques et en ce que l'on décolmate au moins partiellement ledit milieu granualire en la faisant traverser par un second courant du liquide à traiter circulant à une vitesse par unité d'aire de la section transversale supérieure à celle du premier courant et comprise entre 0,25 et 1 m/h, en récupérant en aval du milieu granulaire, dans le sens de passage desdits courants de liquides, des agrégats floculés (4) desdites particules microscopiques et un effluent liquide (6), partiellement ou totalement épuré.

2. Procédé selon la revendication 1, caractérisé en ce qu'une partie au moins de l'effluent liquide (6) est recyclée à l'alimentation dudit milieu granulaire (2).

3. Procédé selon l'une des revendications 1 et 2, caractérisé en ce que ledit milieu granulaire (2) est traversé de bas en haut par ledit premier et ledit second courants du liquide à traiter.

4. Procédé selon la revendication 3, caractérisé en ce que ledit milieu granulaire (2) demeure en lit fixe pendant la mise en oeuvre du procédé.

5. Procédé selon la revendication 3, caractérisé en ce que, pendant la phase de décolmatage, ledit milieu granulaire (2) subit une expansion d'au plus environ 30% en volume.

6. Procédé selon l'une des revendications 1 à 5, caractérisé en ce que ledit milieu granulaire (2) est constitué de particules ayant une granulométrie comprise entre environ 0,005 et 3 cm et une masse volumique d'environ 1,1 g/cm$^3$ ou davantage.

7. Procédé selon la revendication 6, caractérisé en ce que ledit milieu granulaire (2) est constitué d'argile calcinée et broyée de granulométrie comprise entre 3 et 5 mm.

8. Procédé selon l'une des revendications 1 à 7, caractérisé en ce que ledit milieu granulaire (2) est logé dans une colonne (1), verticale ou inclinée, cylindrique ou hélicoïdale, dont la partie (9') située au-dessus dudit milieu (2) a une section plus large que celle contenant ledit milieu.

9. Application du procédé selon l'une des revendications 1 à 8, à l'épuration d'eaux usées.

## Patentansprüche

1. Verfahren zur kontinuierlichen Ausflockung ohne Zusatz eines Reagenz von mikroskopischen, in einer Flüssigkeit schwebenden Teilchen, wie beispielsweise Mikroalgen (d.h. Algen mit Abmessungen zwischen 10 und 30 μm), dadurch gekennzeichnet, daß ein erster, die schwebenden mikroskopischen Teilchen enthaltender Flüssigkeitsstrom über ein granuläres Medium (2) bis zu einer teilweisen oder vollständigen Kolmatierung des granulären Mediums durch die mikroskopischen Teilchen geleitet wird und daß das granuläre Medium zumindest teilweise durch den Durchtritt eines zweiten Flüssigkeitsstromes zur Behandlung gespült wird, wobei der zweite Flüssigkeitsstrom mit einer gegenüber dem ersten Flüssigkeitsstrom größeren Geschwindigkeit pro Querschnittsfläche zirkuliert, welche zwischen 0,25 und 1 m/h liegt, und wobei in Strömungsrichtung der Flüssigkeitsströme gesehen stromabwärts des granulären Mediums ausgeflockte Aggregate (4) der mikroskopischen Teilchen und teilweise oder vollkommen gereinigte abströmende Flüssigkeit (6) rückgewonnen wird.

2. Verfahren nach Anspruch 1, dadurch gekennzeichnet, daß wenigstens ein Teil der abströmenden Flüssigkeit (6) zur Speisung des granulären Mediums (2) rückgeführt wird.

6

3. Verfahren nach Anspruch 1 oder 2, dadurch gekennzeichnet, daß das granuläre Medium (2) von unten nach oben vom ersten Flüssigkeitsttrom und vom zweiten Flüssigkeitsstrom zur Behandlung durchströmt wird.

4. Verfahren nach Anspruch 3, dadurch gekennzeichnet, daß das granuläre Medium (2) während der Durchführung des Verfahrens in einem stationären Bett verbleibt.

5. Verfahren nach Anspruch 3, dadurch gekennzeichnet, daß das granuläre Medium (2) während der Ausspülung einer Ausdehnung von höchstens ungefähr 30 Vol.% unterliegt.

6. Verfahren nach einem der Ansprüche 1 bis 5, dadurch gekennzeichnet, daß das granuläre Medium (2) von Teilchen mit einer Korngröße zwischen 0,005 und 3 cm und einer Dichte von ungefähr 1,1 g/cm$^3$ oder mehr gebildet wird.

7. Verfahren nach Anspruch 6, dadurch gekennzeichnet, daß das granuläre Medium (2) von gebranntem und gemahlenem Ton mit einer Korngröße zwischen 3 und 5 mm gebildet wird.

8. Verfahren nach einem der Ansprüche 1 bis 7, dadurch gekennzeichnet, daß das granuläre Medium (2) in einer vertikalen oder geneigten, zylindrischen oder schraubenförmigen Kolonne (1) angeordnet ist, deren oberhalb des Mediums (2) angeordneter Abschnitt (9') einen größeren Querschnitt als jenen des das Medium enthaltenden Abschnittes aufweist.

9. Anwendung des Verfahrens nach einem der Ansprüche 1 bis 8 zur Abwasserreinigung.

**Claims**

1. A process for the continuous flocculation, without the addition of reagents, of microscopic particles such as micro-algae (that is to say algae of dimensions of between 10 and 30 µm) in suspension in a liquid, characterized in that a first stream of the liquid comprising the microscopic particles in suspension is passed through a granular medium (2) until said granular medium is partially or completely clogged up by said microscopic particles and in that said granular medium is cleaned at least partially by sending through it a second stream of the liquid to be treated moving at a speed per unit area of the transverse section higher than that of the first stream and of between 0.25 and 1 m/h, and recovering downstream of the granular medium, in the direction of travel of said streams of liquid, flocculated aggregates (4) of said microscopic particles and a partially or totally purified liquid effluent (6).

2. A process according to claim 1, characterized in that at least part of the liquid effluent (6) is recycled to the supply line of said granular medium (2).

3. A process according to claim 1 or claim 2, characterized in that said first and said second strams of liquid to be treated are passed through said granular medium (2) in an upwards direction.

4. A process according to claim 3, characterized in that said granular medium (2) remains as a fixed bed during implementation of the process.

5. A process according to claim 3, characterized in that, during the cleaning phase, said·granular medium (2) undergoes an expansion of at most approximately 30% in volume.

6. A process according to any one of claims 1 to 5, characterized in that said granular medium (2) consists of particles of a granular size of between approximately 0.005 and 3 cm and a density of approximately 1.1 g/cm$^3$ or more.

7. A process according to claim 6, characterized in that said granular medium (2) consists of calcined, crushed clay of a granular size of between 3 and 5 mm.

8. A process according to any one of claims 1 to 7, characterized in that said granular medium (2) is located in a vertical or inclined, cylindrical or helical column (1), of which the part (9') situated above said medium (2) has a wider section than that containing said medium.

9. The use of the process according to any one of claims 1 to 8 for purifying waste water.

FIG.1

FIG.2

FIG.3